# DEMANDE DE BREVET EUROPEEN

(11) **EP 1 757 262 A1**
(43) Date de publication de la demande: **28.02.2007**
(21) Numéro de dépôt: 06118545.0
(22) Date de dépôt: 07.08.2006
(51) Int. Cl.: A61K 8/19, A61K 8/31, A61K 8/04, A61K 8/81, A61Q 1/10, A61Q 1/12

(54) **Fard à paupières compacté comprenant au moins 50% en poids de matières colorantes**

(30) Priorité: 26.08.2005 FR 0552575
(71) Demandeur: L'ORÉAL, 75008 Paris (FR)
(72) Inventeur: Bouchard, Fabienne, 94800, Villejuif (FR)
(74) Mandataire: Duvert, Sandra

(57) **Abrégé**

L'invention a pour objet une composition de fard à paupières comprenant au moins 50% en poids par rapport au poids total de la composition, d'au moins un agent de coloration produisant une couleur par absorption d'au moins une partie du spectre visible et une phase grasse solide comprenant une eire.

## Description

La présente invention a pour objet une composition cosmétique de fard-à-paupières comprenant un taux élevé de matières colorantes. L'invention a aussi pour objet un procédé de maquillage des paupières utilisant la composition.

Les fards à paupières sont constituées d'un véhicule approprié et de différentes matières colorantes destinées à conférer une certaine couleur au fard et après leur application sur les paupières. Ils peuvent se présenter sous forme aqueuse ou anhydre.

De nombreuses compositions de maquillage de ce type se présentent sous forme de poudre compacte comprenant généralement une phase grasse, appelée classiquement « liant », et une phase pulvérulente comprenant notamment des pigments et/ou des charges. Le liant a pour fonction principale de garantir une cohésion suffisante de la composition finale, notamment de manière a lui éviter une fragmentation pouvant être provoquée par les chocs et de lui conférer par ailleurs une bonne aptitude au prélèvement.
L'obtention de ces propriétés implique par ailleurs que les autres composants de la composition cosmétique, et en particulier les pigments qu'elle contient s'avèrent également appropriées pour une mise en forme de type poudre compacte.

On cherche à obtenir des fards à paupières présentant un effet coloriel intense, en particulier par l'incorporation d'un taux élevé de matières colorantes tels que des pigments minéraux ou organiques.
Toutefois, les pigments conventionnels tels que par exemple les oxydes de fer présentent une bonne aptitude au compactage, pouvant se traduire par une trop forte cohésion du produit, rendant difficile son délitage à l'aide d'un applicateur tel qu'une houpette ou un pinceau, ou bien au doigt.
Par conséquent, l'incorporation de matière colorante à un taux élevée est limitée par l'exigence de garder une bonne aptitude au prélèvement du produit.

Le but de la présente invention est de disposer d'un fard à paupières se présentant sous forme d'une poudre compacte comprenant une taux élevé de matières colorantes et présentant une couleur intense, ayant une bonne cohésion, une bonne résistance aux chocs et une bonne aptitude au prélèvement.

La présente invention a donc pour objet un fard à paupières sous forme de poudre compacte comprenant au moins 50% en poids par rapport au poids total de la composition, d'au moins un agent de coloration produisant une couleur par absorption d'au moins une partie du spectre visible et une phase grasse solide comprenant au moins un composé choisi parmi les cires..

Une couleur produite par absorption de la lumière est encore qualifiée parfois de couleur chimique, par opposition aux couleurs produites par un phénomène d'interférences, y compris de diffraction, encore appelées couleurs physiques. Le phénomène d'absorption d'énergie lumineuse par l'agent de coloration peut reposer sur des transitions électroniques.

L'invention a également pour objet l'utilisation, dans un fard à paupière, d'au moins 50% en poids par rapport au poids total de la composition, d'au moins un agent de coloration produisant une couleur par absorption d'au moins une partie du spectre visible pour obtenir un fard à paupières présentant une couleur intense, ayant une bonne cohésion, une bonne résistance aux chocs et une bonne aptitude au prélèvement.

L'invention a également pour objet un procédé cosmétique de maquillage des paupières comprenant l'application sur les paupières, d'un fard à paupières tel que défini précédemment.

### Agent de coloration

L'agent de coloration produisant une couleur par un phénomène d'absorption peut être constitué par un pigment organique ou inorganique ou hybride comportant à la fois de la matière organique et de la matière inorganique.
L'agent de coloration peut être un composé particulaire ou non.
Lorsque l'agent de coloration comporte un colorant, celui-ci peut être choisi parmi les colorants liposolubles et hydrosolubles.
Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.
Les colorants hydrosolubles sont par exemple le jus de betterave et le bleu de méthylène.

Les colorants peuvent par exemple représenter de 0,1 à 20 % du poids de la première ou de la deuxième composition, voire de 0,1 à 6 %, lorsque présents.
L'agent de coloration peut encore être une laque ou un pigment organique choisi parmi les matériaux ci-dessous et leurs mélanges :
- le carmin de cochenille,
- les pigments organiques de colorants azoïques, anthraquinoniques, indigoïdes, xanthéniques, pyréniques, quinoliniques, de triphénylméthane, de fluorane,
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.
Parmi les pigments organiques, on peut notamment citer les pigments certifiés D&C connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.
L'agent de coloration peut être une laque organique supportée par un support organique tel que la colophane ou le benzoate d'aluminium, par exemple.
Parmi les laques organiques, on peut en particulier citer les laques certifiées D&C connues sous les dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.
Les matériaux chimiques correspondant à chacune des matières colorantes organiques citées précédemment sont mentionnés dans l'ouvrage « International Cosmetic Ingredient Dictionnary and Handbook », Edition 1997, pages 371 à 386 et 524 à 528, publié par « The Cosmetic, Toiletry, and Fragrance Association », dont le contenu est incorporé dans la présente demande par référence.
L'agent de coloration peut être un pigment composite, comportant un noyau enrobé au moins partiellement par une écorce.

### Pigments composites

Un pigment composite selon l'invention peut être composé notamment de particules comportant :
- un noyau inorganique,
- au moins un enrobage au moins partiel d'au moins une matière colorante organique.

Au moins un liant peut avantageusement contribuer à la fixation de la matière colorante organique sur le noyau inorganique.

Les particules de pigment composite peuvent présenter des formes variées. Ces particules peuvent être notamment en forme de plaquettes ou globulaires, en particulier sphériques, et être creuses ou pleines. Par « en forme de plaquettes », on désigne des particules dont le rapport de la plus grande dimension à l'épaisseur est supérieur ou égal à 5.

Un pigment composite peut présenter par exemple une surface spécifique comprise entre 1 et 1000 m²/g, notamment entre 10 et 600 m²/g environ, et en particulier entre 20 et 400 m²/g environ. La surface spécifique est la valeur mesurée par la méthode BET.

La proportion massique du noyau peut excéder 50 % par rapport au poids total du pigment composite, par exemple aller de 50 % à 70 %, par exemple de 60 % à 70 %.

Le pigment composite peut être différent d'un pigment interférentiel tel que décrit par exemple dans le brevet US 6 428 773. Un pigment interférentiel comporte par exemple plusieurs couches d'épaisseurs constantes de matériaux sélectionnés pour pouvoir produire des interférences optiques.

La saturation C* du pigment composite peut être supérieure ou égale à 30, mesurée selon le protocole ci-dessous.

### Protocole de mesure de la saturation du pigment composite :

Les valeurs a* et b* dans l'espace CIE L*a*b* du pigment composite sont mesurées comme suit :

Le pigment composite pur est compacté dans une coupelle rectangulaire ayant pour dimensions 2 x 1,5 cm et une profondeur de 3 mm, en appliquant une pression de 100 bars. Les valeurs a* et b* du pigment compacté sont mesurées avec un spectrophotomètre MINOLTA 3700d, en mode spéculaire exclu, sous illuminant D65, ouverture moyenne. La saturation est donnée par C* = (a^{*2} + b^{*2})^{1/2}.

### Noyau inorganique

Le noyau inorganique peut être de toute forme convenant à la fixation de particules de matière colorante organique, par exemple sphérique, globulaire, granulaire, polyédrique, aciculaire, fusiforme, aplatie en forme de flocon, de grain de riz, d'écaille, ainsi qu'une combinaison de ces formes, cette liste n'étant pas limitative.

De préférence, le rapport de la plus grande dimension du noyau à sa plus petite dimension est compris entre 1 et 50.

Le noyau inorganique peut présenter une taille moyenne comprise entre environ 1 nm et environ 100 nm, voire entre environ 5 nm et environ 75 nm, par exemple entre environ 10 nm et environ 50 nm, notamment 20 ou 25 nm.

Par « taille moyenne », on désigne la dimension donnée par la distribution granulométrique statistique à la moitié de la population, dite D50. La taille moyenne peut être une taille moyenne en nombre déterminée par analyse d'image (microscopie électronique).

Le noyau inorganique peut présenter un indice de réfraction supérieur ou égal à 2, voire supérieur ou égal à 2,1, par exemple supérieur ou égal à 2,2.

Le noyau inorganique peut être réalisé dans un matériau magnétique ou non choisi dans la liste non limitative comprenant les sels métalliques et oxydes métalliques, notamment les oxydes de titane, de zirconium, de cérium, de zinc, de fer, de bleu ferrique, d'aluminium et de chrome, les alumines, les verres, les céramiques, le graphite, les silices, les silicates, notamment les aluminosilicates et les borosilicates, le mica synthétique, et leurs mélanges.

Les oxydes de titane, notamment TiO₂, de fer, notamment Fe₂O₃, de cérium, de zinc et d'aluminium, les silicates, notamment les aluminosilicates et les borosilicates conviennent tout particulièrement.

Le noyau inorganique peut présenter une surface spécifique, mesurée par la méthode BET, comprise par exemple entre environ 1 m²/g et environ 1000 m²/g, mieux entre enviLe noyau inorganique peut être coloré, le cas échéant.

### Matière colorante organique

La matière colorante organique peut comporter par exemple au moins un pigment organique, par exemple au moins une laque organique.

La matière colorante organique peut être choisie par exemple parmi les composés particulaires insolubles dans le milieu physiologiquement acceptable de la composition.

La matière colorante organique peut comporter par exemple des pigments, par exemple des laques organiques ou autres matières colorantes organiques, qui peuvent être choisis parmi les laques ou pigments organiques cités plus haut

La proportion massique de matière colorante organique peut être comprise entre environ 10 parts et environ 500 parts en poids pour 100 parts du noyau inorganique, voire entre environ 20 parts et environ 250 parts en poids, par exemple entre environ 40 parts et environ 125 parts en poids pour 100 parts du noyau inorganique.

La proportion de la matière colorante organique peut excéder 30 % par rapport au poids total du pigment composite, par exemple aller de 30 à 50 %, par exemple de 30 à 40 %.

### Liant du pigment composite

Le liant du pigment composite peut être de tout type dès lors qu'il permet à la matière colorante organique d'adhérer à la surface du noyau inorganique.

Le liant peut notamment être choisi parmi une liste non limitative comprenant les composés siliconés, les composés polymériques, oligomériques ou similaires, et en particulier parmi les organosilanes, les organosilanes fluoroalkylés et les polysiloxanes, par exemple le polyméthylhydrogénosiloxane, ainsi que divers agents couplants, tels que des agents couplants à base de silanes, de titanates, d'aluminates, de zirconates et leurs mélanges.

Le composé siliconé peut être choisi parmi une liste non limitative comprenant notamment :
- les organosilanes (1) obtenus à partir d'alkoxysilanes,
- les polysiloxanes (2) modifiés ou non choisis parmi une liste non limitative comprenant :
   - les polysiloxanes modifiés (2A) comprenant au moins un radical choisi parmi, notamment, les polyéthers, les polyesters et les composés époxy (ils seront appelés « polysiloxanes modifiés »),
   - les polysiloxanes (2B) portant sur un atome de silicium situé à l'extrémité du polymère, au moins un groupe choisi parmi une liste non limitative comprenant les acides carboxyliques, les alcools ou les groupes hydroxy, et
- les composés organosilanes fluoroalkylés (3) obtenus à partir de fluoroalkylsilanes.

Les composés servant de liant peuvent notamment présenter une masse molaire pouvant varier entre 300 et 100 000.

cPour obtenir une couche recouvrant les noyaux inorganiques uniformément, le liant est de préférence dans un état liquide ou soluble dans l'eau ou dans différents solvants.

La quantité de liant peut varier de 0,01 à 15 %, notamment de 0,02 à 12,5 % et en particulier de 0,03 à 10% en poids (calculée par rapport à C ou Si) par rapport au poids des particules comprenant le noyau et le liant. Pour de plus amples détails sur la manière de calculer la quantité relative du liant, on pourra se reporter à la demande EP 1 184 426 A2. La proportion relative de liant peut être inférieure ou égale à 5 %, par exemple inférieure ou égale à 3 %, par rapport au poids total du pigment composite.

Le pigment composite peut être réalisé par exemple par l'un des procédés décrits dans les demandes de brevet européen EP 1 184 426 et EP 1 217 046, dont les contenus sont incorporés ici par référence, avantageusement par le procédé décrit dans la demande EP 1 184 426.

Une autre méthode de fabrication d'un pigment composite est décrite dans le brevet JP 3286463, qui divulgue un procédé de précipitation en solution.

De préférence, l'agent de coloration est choisi parmi :
les pigments organiques tels que
   - le noir de carbone,
   - les pigments certifiés D & C par la Food & Drug Administration tels que répertoriés à la section « Color Additives - Batch Certified by the U.S. Food and Drug Administration » du CTFA ; on peut citer notamment les Blue 1 et 4, Brown 1, Ext.Violet 2, Ext.Yellow 7, les Green 3, 5, 6, 8, les Orange 4, 5, 10, 11, les Red 4, 6, 7, 17, 21, 22, 27, 28, 30, 36 et 40, le Violet 2, les Yellow 5, 6, 7, 8, 10 et 11,
   - les laques à base de carmin de cochenille, de baryum, strontium, calcium, aluminium,
les pigments minéraux tels que :
   - les oxydes de fer, de titane, de zirconium, de cérium, de zinc, de fer ou de chrome,
   - le bleu ferrique, le violet de manganèse, le bleu, le rose ou le violet d'outremer, l'hydrate de chrome, l'hydroxyde de chrome, l'oxychlorure de bismuth,
   - et leurs mélanges.

Le fard à paupières selon l'invention comprend la ou les matière(s) colorante(s) en une teneur supérieure ou égale à 50 % en poids, par rapport au poids total de la composition, par exemple allant de 50 à 90% en poids, de préférence en une teneure supérieure ou égale à 55% en poids, allant par exemple de 55 à 80% en poids, et mieux supérieure ou égale à 60% en poids, par exemple de 60 à 70%.

### Phase grasse solide :

La composition selon l'invention comporte au moins une phase grasse solide, dite encore « liant sec ou solide». La phase grasse solide comprend au moins un composé choisi parmi les cires.

Par « liant solide », on entend au sens de la présente invention une phase grasse dont le point de fusion peut être supérieur ou égal à 30 °C, notamment peut aller de 30 à 250 °C et en particulier de 30 à 230 °C.

Par "cire", au sens de la présente invention, on entend un composé gras lipophile, solide à température ambiante (25°C) et pression atmosphérique (760 mm Hg, soit 10⁵ Pa), à changement d'état solide/liquide réversible, ayant en particulier une température de fusion supérieure ou égale à 30°C, notamment supérieure ou égale à 55 °C, et pouvant aller jusqu'à 250 °C, notamment jusqu'à 230 °C, et en particulier jusqu'à 120 °C.

En portant la cire à sa température de fusion, il est possible de la rendre miscible aux huiles et de former un mélange homogène microscopiquement, mais en rétablissant la température du mélange à la température ambiante, on obtient une recristallisation de la cire dans les huiles du mélange.

Les valeurs de point de fusion correspondent, selon l'invention, au pic de fusion mesurée à l'aide d'un calorimètre à balayage différentiel (D.S.C.), par exemple le calorimètre vendu sous la dénomination DSC 30 par la société METLER, avec une montée en température de 5 ou 10 °C par minute.

Les cires, au sens de l'invention, peuvent être celles utilisées généralement dans les domaines cosmétiques ou dermatologiques. Elles peuvent notamment être hydrocarbonées, siliconées et/ou fluorées, comportant éventuellement des fonctions ester ou hydroxyle. Elles peuvent être également d'origine naturelle ou synthétique.

A titre illustratif et non limitatif de ces cires, on peut notamment citer :
- la cire d'abeille, la cire de lanoline, et les cires d'insectes de Chine ; la cire de riz, la cire de Carnauba, la cire de Candellila, la cire d'Ouricury, la cire de fibres de liège, la cire de canne à sucre, la cire du Japon et la cire de sumac; la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides gras et les glycérides concrets à 40 °C et notamment à plus de 55 °C,
- les cires obtenues par hydrogénation catalytique d'huiles animales ou végétales ayant des chaînes grasses, linéaires ou ramifiées, en C₈-C₃₂, notamment l'huile de jojoba hydrogénée, l'huile de tournesol hydrogénée, l'huile de ricin hydrogénée, l'huile de coprah hydrogénée et l'huile de lanoline hydrogénée,
- les cires de silicones ou les cires fluorées, et
- leurs mélanges.

De préférence, la phase grasse solide comprend au moins une cire présente totalement ou partiellement sous forme de poudre, notamment micronisée.

De préférence, la cire est choisie parmi les cires de carnauba, les cires de paraffine et leurs mélanges.

Parmi les cires utilisables sous forme de poudre, on peut notamment citer les microbilles de cire de Carnauba vendues sous la dénomination Microcare 350^{®} par la société Micro Powders et les microbilles de cire de paraffine vendues sous la dénomination Microease 114S^{®} par la société Micro Powders.

Cette phase grasse solide peut comprendre au moins un composé les savons métalliques.

Parmi ceux ci, on peut notamment citer les savons métalliques d'acides gras ayant de 12 à 22 atomes de carbone, et en particulier ceux ayant de 12 à 18 atomes de carbone.

Le métal du savon métallique peut notamment être du zinc ou du magnésium.

L'acide gras peut notamment être choisi parmi l'acide laurique, l'acide myristique, l'acide stéarique, l'acide palmitique.

Comme savon métallique, on peut utiliser le laurate de zinc, le stéarate de magnésium, le myristate de magnésium, le stéarate de zinc, et leurs mélanges.

Selon une variante particulière de l'invention, la phase grasse solide peut comprendre au moins un savon métallique présent totalement ou partiellement sous forme de poudre.

La phase grasse solide peut être présente en une teneur allant de 1% à 30% en poids, notamment de 2% à 20% en poids par rapport au poids total de la composition.

### Phase grasse liquide :

La composition selon l'invention peut en outre comprendre une phase grasse liquide (aussi appelée liant gras) comprenant au moins une huile.

Cette huile peut être choisie parmi les huiles utilisées classiquement comme liant dans les poudres compactes.

Ces huiles peuvent être notamment choisies parmi :
- l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide ;
- les huiles d'hydrocarbures, telles que les huiles de paraffine, le squalane, la vaseline, le polydécène, notamment hydrogéné, comme le « CERAFLOW E^{®} » commercialisé par la société SHAMROCK ;
- les esters gras, tels que le myristate d'isopropyle, le palmitate d'isopropyle, le stéarate de butyle, le stéarate d'isodécyle, le laurate d'hexyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine
- les huiles de silicone telles que les polyméthylsiloxanes, les polyméthylphénylsiloxanes, les polysiloxanes modifiés par des acides gras, des alcools gras ou des polyoxyalkylènes, les silicones fluorées ou les huiles perfluorées ;
- les acides gras supérieurs tels que l'acide myristique, l'acide palmitique, l'acide stéarique, l'acide béhénique, l'acide oléique, l'acide linoléique, l'acide linolénique ou l'acide isostéarique;
- les alcools gras supérieurs tels que le cétanol, l'alcool stéarylique ou l'alcool oléique ;
- leurs mélanges.

Selon un mode avantageux de réalisation, le fard à paupières selon l'invention comprend une phase grasse liquide en une teneur inférieure ou égale à 10% en poids par rapport au poids total de la composition, de préférence inférieure ou égale à 7% en poids, de préférence inférieure ou égale à 5% en poids, mieux inférieure ou égale à 3% en poids et encore mieux inférieure ou égale à 3% en poids ; encore mieux la composition de fard à paupière est exempte de phase grasse liquide. Ceci permet de limiter les phénomènes de « cirage », on dit que le produit cire lorsqu'il est trop compact et difficile à prélever.

### Charges

Le fard à paupières selon l'invention peut comprendre au moins une charge qui peut être organique ou minérale, de forme sphérique ou lamellaire.

Les charges peuvent être choisies parmi les charges dites incompactables telles que :
- les microsphères de silice, notamment à porosité ouverte ou, de préférence, les microsphères de silice creuses, telles que les "SILICA BEADS SB 700/HA" ou "SILICA BEADS SB 700" de la société MAPRECOS, ; ces microsphères peuvent être imprégnées d'un actif cosmétique ;
- les microsphères microporeuses de polymères, qui ont une structure analogue à celle d'une éponge; elles ont, en général, une surface spécifique d'au moins 0,5 m²/g et, en particulier, d'au moins 1 m²/g, ladite surface spécifique n'ayant pas de limite supérieure autre que celle résultant de la possibilité pratique de réaliser des microsphères de porosité très élevée : la surface spécifique peut, par exemple, atteindre 1 000 m²/g ou même davantage. On peut citer les microsphères de polymères acryliques, telles que celles en copolymère d'acrylate réticulé 'Polytrap 6603 Adsorber' de la société RP SCHERER, et celles de polyméthacrylate de méthyle 'MICROPEARL M 100' de la société SEPPIC;
- la poudre de polyuréthane telle que la poudre de copolymère de diisocyanate d'hexaméthylène et de triméthylol hexyl lactone vendue sous les dénomination PLASTIC POWDER D-400 et T-7 par la société TOSHIKI;
- les microcapsules de polymères qui comportent une seule cavité fermée et forment un réservoir, qui peut contenir un liquide, notamment un actif cosmétique; elles sont préparées par des procédés connus tels que ceux décrits dans le brevet US-A 3 615 972 et EP-A 0 56219 . Elles peuvent être réalisées, par exemple, en polymères ou copolymères d'acides, d'amines ou d'esters monomères à insaturation éthylénique, en polymères uréeformaldéhyde, en polymères ou copolymères de chlorure de vinylidène; à titre d'exemple, on peut citer les microcapsules faites de polymères ou copolymères d'acrylate ou de méthacrylate de méthyle, ou encore de copolymères de chlorure de vinylidène et d'acrylonitrile; parmi ces derniers, on indiquera, notamment, ceux qui contiennent, en poids 20-60% de motifs dérivés de chlorure de vinylidène, 20-60% en poids de motifs dérivés d'acrylonitrile et 0-40% en poids d'autres motifs tels que des motifs dérivés d'un monomère acrylique et/ou styrénique; on peut également utiliser des polymères ou copolymères acryliques réticulés ;
- les poudres sphériques d'organopolysiloxane réticulés élastomères, notamment décrites dans le document JP-A-02-243612, telles que celles vendues sous la dénomination "TREFIL POWDER E-506C" par la société DOW CORNING.

La composition peut contenir, en plus des charges incompactables, des charges dites compactables.

Comme charges compactables de forme lamellaire, on peut citer :
- les talcs ou silicates de magnésium hydratés, notamment sous forme de particules de dimensions généralement inférieures à 40 µm;
- les micas ou aluminosilicates de compositions variées et qui se présentent notamment sous forme d'écailles ayant des dimensions de 2 à 200 µm, de préférence 5-70 µm et une épaisseur de 0,1 à 5 µm, de préférence de 0,2-3 µm, ces micas pouvant être d'origine naturelle (par exemple muscovite, margarite, roscoelite, lipidolite, biotite) ou d'origine synthétique ;
- les argiles telles que les séricites, qui appartiennent à la même classe chimique et cristalline que la muscovite ;
- le kaolin ou silicate d'aluminium hydraté, qui se présente notamment sous la forme de particules de formes isotropes ayant des dimensions généralement inférieures à 30µm ;
- les nitrures de bore,
- les poudres de polymères de tétrafluoroéthylène, telles que le 'CERIDUST 9205 F' de la société CLARIANT
- le carbonate de calcium précipité, notamment sous forme de particules de dimensions supérieures à 10 µm ;
- le carbonate et l'hydrocarbonate de magnésium ;
- l'hydroxyapatite.
- les poudres de polymères synthétiques non expansés, tels que le polyéthylène, les polyesters (par exemple isophtalate ou téréphtalate de polyéthylène) et les polyamides (par exemple le Nylon), sous la forme de particules ayant des dimensions inférieures à 50 µm ;
- les poudres de polymères synthétiques, réticulés ou non, sphéronisées, comme les poudres de polyamides telles que les poudres de poly-β-alanine ou de Nylon, par exemple la poudre 'Orgasol' de la société ATOCHEM, des poudres d'acide polyacrylique ou polyméthacrylique, des poudres de polystyrène réticulé par le divinylbenzène et des poudres de résine de silicone, et
- des poudres de matériaux organiques d'origine naturelle comme les amidons, notamment de maïs, de blé ou de riz.
et leurs mélanges

Les charges peuvent être présentes dans la composition en une teneur allant de 0,1 % à 50% en poids par rapport au poids total de la composition, de préférence allant de 1% à 40% en poids.

Le fard à paupières selon l'invention peut comprendre, outre les agents de coloration citées plus haut, un agent de coloration additionnel, choisi parmi les pigments nacrés qui peuvent être choisis parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

La composition peut comporter au moins un pigment interférentiel ou diffractant, et/ou des particules réfléchissantes.

On peut citer les pigments à effet tels les particules comportant un substrat organique ou minéral, naturel ou synthétique, par exemple le verre, les céramiques, les alumines, les silices, les silicates et notamment aluminosilicates et les borosilicates, le mica synthétique tel que le fluorophlogopite, les résines acrylique, le polyester, le polyuréthane, le polyéthylène téréphtalate, les métaux et les dérivés métalliques et leurs mélanges, cette liste n'étant pas limitative, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

On peut également utiliser les agents de coloration goniochromatiques, les pigments interférentiels, notamment à cristaux liquides ou multicouches, les agents thermo-chromes, les agents azurants optiques.

La composition selon l'invention est avantageusement une composition anhydre, c'est-à-dire une composition contenant moins de 2 % en poids d'eau, voire moins de 0,5 % d'eau, notamment moins de 0,2% d'eau" l'eau n'étant pas ajoutée lors de la préparation de la composition mais correspondant à l'eau résiduelle apportée par les ingrédients mélangés.

La composition peut comprendre d'autres ingrédients (adjuvants) usuellement utilisés en cosmétique tels que les conservateurs, les actifs cosmétiques, les agents hydratants, les filtres UV, les épaississants, les tensioactifs, les parfums.

Bien entendu, l'homme de l'art veillera à choisir le ou les éventuels adjuvants ajoutés à la composition selon l'invention de manière telle que les propriétés avantageuses attachées intrinsèquement à la composition conforme à l'invention ne soient pas, ou substantiellement pas, altérées par l'addition envisagée.

La composition peut être préparée en mélangeant les ingrédients de la phase pulvérulentes (charges et pigments) puis en ajoutant la phase grasse sous agitation, le mélange étant ensuite broyé et/ou mélangé vigoureusement, éventuellement tamisé, puis versé dans une coupelle et compacté, par exemple à l'aide d'une presse.
La composition ainsi obtenue se présente sous forme de poudre compacte.

La présente invention est illustrée plus en détail dans les exemples suivants :

### Exemple 1:

On a préparé le fard à paupières compacté suivant :

| | |
|---|---|
| Oxydes de fer noir | 60 g |
| Ultramarines and silica | 0,5 g |
| Talc | 25,7g |
| Stéarate de magnésium | 8 g |
| Microbilles de cire de paraffine (Microease ^{®} 114S de Micro Powders) | 5 g |
| Polydécène hydrogéné (Ceraflow E^{®} de Shamrock ) | 3,00 g |
| Conservateurs | 0,8 g |

La composition compactée a une bonne résistance aux chocs et se délite facilement à l'aide d'un applicateur.

## Revendications

1. Fard à paupières sous forme de poudre compacte comprenant au moins 50% en poids par rapport au poids total de la composition, d'au moins un agent de coloration produisant une couleur par absorption d'au moins une partie du spectre visible et une phase grasse solide comprenant au moins un composé choisi parmi les cires.

2. Fard à paupières selon la revendication 1, **caractérisé par le fait que** l'agent de coloration est présent en une teneur allant de 50 à 90% en poids, de préférence allant de 55 à 80% en poids, et mieux de 60 à 70% en poids par rapport au poids total de la composition.

3. Fard à paupières selon la revendication 1, **caractérisé par le fait que** l'agent de coloration est présent en une teneur supérieure ou égale à 55 % en poids, de préférence supérieure ou égale à 60% en poids par rapport au poids total de la composition.

4. Fard à paupières selon l'une des revendications 1 à 3, **caractérisé par le fait que** l'agent de coloration est choisi parmi :
- les pigments organiques tels que
- le noir de carbone,
- les pigments certifiés D & C et notamment les Blue 1 et 4, Brown 1, Ext.Violet 2, Ext.Yellow 7, les Green 3, 5, 6, 8, les Orange 4, 5, 10, 11, les Red 4, 6, 7, 17, 21, 22, 27, 28, 30, 36 et 40, le Violet 2, les Yellow 5, 6, 7, 8, 10 et 11,
- les laques à base de carmin de cochenille, de baryum, strontium, calcium, aluminium,
- les pigments minéraux tels que :
- les oxydes de fer, de titane, de zirconium, de cérium, de zinc, de fer ou de chrome,
- le bleu ferrique, le violet de manganèse, le bleu, le rose ou le violet d'outremer, l'hydrate de chrome, l'hydroxyde de chrome, l'oxychlorure de bismuth,
- et leurs mélanges.

5. Fard à paupières selon l'une des revendications précédentes, **caractérisé par le fait que** la phase grasse solide comprend au moins un composé choisi parmi les savons métalliques et leurs mélanges.

6. Fard à paupières selon l'une des revendications précédentes, **caractérisé par le fait que** la cire est présente totalement ou partiellement sous forme de poudre.

7. Fard à paupières selon l'une des revendications précédentes, **caractérisé par le fait que** la cire est une cire de paraffine ou de carnauba.

8. Fard à paupières selon l'une des revendications précédentes, **caractérisé par le fait que** la phase grasse solide représente de 1% à 30% en poids, notamment de 2% à 20% en poids par rapport au poids total de la composition.

9. Fard à paupières selon l'une des revendications précédentes, **caractérisé par le fait qu'**il comprend au moins une charge.

10. Fard à paupières selon la revendication 9, **caractérisé par le fait que** la charge est présente en une teneur allant de 0,1 % à 50% en poids par rapport au poids total de la composition, de préférence allant de 1 % à 40% en poids.

11. Fard à paupières selon l'une des revendications précédentes, **caractérisé par le fait qu'**il comprend une phase grasse liquide en une teneur inférieure ou égale à 10% en poids par rapport au poids total du fard à paupières.

12. Fard à paupières selon l'une quelconque des revendications précédentes, **caractérisé par le fait qu'**il comprend un ingrédient cosmétique choisi parmi les conservateurs, les actifs cosmétiques, les agents hydratants, les filtres UV, les épaississants, les tensioactifs, les parfums.

13. Procédé cosmétique de maquillage des paupières comprenant l'application sur les paupières d'un fard à paupières selon l'une quelconque des revendications précédentes.
